# EUROPEAN PATENT SPECIFICATION

(11) **EP 3 806 928 B1**
(45) Date of publication and mention of the grant of the patent: **30.07.2025**
(21) Application number: 19726706.5
(22) Date of filing: 29.05.2019
(51) Int. Cl.: A61M 5/142, A61M 5/168, A61M 39/28

(54) **INFUSION DEVICE COMPRISING A CLAMPING MECHANISM**
INFUSIONSVORRICHTUNG MIT EINEM KLEMMMECHANISMUS
DISPOSITIF DE PERFUSION COMPRENANT UN MÉCANISME DE SERRAGE

(30) Priority: 18.06.2018 EP 18305742
(43) Date of publication of application: 21.04.2021
(73) Proprietor: Fresenius Vial SAS, 38590 Brézins (FR)
(72) Inventor: ARCHAT, Damien, 38000 Grénoble (FR); PAOLI, Mathieu, 38140 La Murette (FR)
(74) Representative: Fresenius Kabi Deutschland GmbH
(86) International application number: PCT/EP2019/063932
(87) International publication number: WO 2019/243010

(56) References cited:
- WO-A1-2017/063930
- WO-A1-93/05829
- US-A1- 2007 265 559
- US-A1- 2010 040 481

## Description

The invention relates to an infusion device according to the preamble of claim 1.

An infusion device of this kind comprises a housing having a receptacle for receiving an infusion line, a pump mechanism for delivering a medical fluid through an infusion line received in the receptacle, and a clamping mechanism having a clamping element being pivotably connected to the housing and being movable with respect to the housing between a clamping position for acting onto an infusion line received in the receptacle to locally pinch off the infusion line and a release position for allowing a flow through the infusion line.

An infusion device, in particular a volumetric (peristaltic) infusion device, serves to deliver a medical fluid, such as a medication or a nutritional solution for example for the enteral feeding, to a patient and for this, via its pump mechanism, acts onto an infusion line. The infusion line on one end may be connected for example to a container containing the medical solution and on its other end to the patient, the pump mechanism exerting a pumping force for example in a peristaltic fashion on the infusion line such that the medical solution is transported through the infusion line towards the patient.

For delivering a medical solution to the patient, an infusion set comprising the infusion line is placed on the infusion device, the infusion line being received in the receptacle of the infusion device. Herein, when placing an infusion line on an infusion device it is to be avoided that a medical solution flows through the infusion line towards the patient in an uncontrolled fashion (so-called free flow condition). For this, the infusion device comprises a clamping mechanism which shall stop a flow through the infusion line at the closing of a door of the infusion device (prior to the start of the actual infusion operation), which also allows to perform an integrity check, in particular a so-called OCS (occlusivity check system) test with which the correct functioning of the system, in particular the pumping mechanism is tested. For starting the infusion operation the clamping mechanism is opened such that a flow through the infusion line may take place controlled by the pump mechanism.

In an infusion device known from US 2010/0040481 A1 a slide clamp acts onto an infusion line, the slide clamp being automatically closed for pinching off the infusion line when closing a door of the infusion device. For opening the slide clamp for conducting an infusion operation an opening mechanism by means of a finger acts onto the slide clamp, such that the pinching of the infusion line is released and a flow through the infusion line may take place.

In WO 2017/063930 A1 a medical pump is disclosed comprising a holder for a hose clamp, wherein the hose clamp is separate from the pump. The hose clamp comprises two clamp portions movable relative to each other in order to clamp the hose due to a rotational movement of a second clamp portion relative to a first clamp portion. The hose is pinched between a rotative part and a fixed part.

Similar to this, the US2007265559 A1 refers to a transfusion safety device having a clamping mechanism, wherein a pair of arms of the clamping mechanism act as a scissor to pinch a tube.

The apparatus of WO 93/05829 A1 discloses a dedicated clamp mechanism distinct from the housing of an infusion device, wherein the clamp mechanism is a symmetrical sliding clamp, pinching a tube thanks to a elastic deformation of the slide clamp.

It is desirable to provide an infusion device which is compatible with different infusion lines of different manufacturers. It furthermore is desirable to provide an infusion device having a clamping mechanism of simple structure and constitution, which is easy and intuitive to use for a user, in particular a nurse in a hospital environment and which furthermore provides for a reliable pinching of an infusion line to prevent a free flow condition even after opening the door of the infusion device.

It is an object of the instant invention to provide an infusion which allows to provide for an easy and intuitive use for placing an infusion line on the infusion device, which may allow the use of different infusion lines on the infusion device and which enables a reliable pinching of the infusion line in order to prevent a free flow condition.

This object is achieved by means of an infusion device comprising the features of claim 1.

Accordingly, the clamping mechanism comprises a base element arranged on the housing, the clamping element being movable with respect to the base element for sliding the clamping element onto the infusion line for pinching the infusion line in between a first clamping face of the clamping element and a second clamping face of the base element in the clamping position or for sliding the clamping element off the infusion line for allowing a flow through the infusion line in the release position.

The clamping mechanism hence comprises a base element which in principle is arranged stationary on the housing and which is not (actively) moved for transferring the clamping mechanism in between the clamping position and the release position. The clamping mechanism furthermore comprises a clamping element which is movable with respect to the base element and establishes a clamping of the infusion line in the clamping position by pinching off the infusion line in between a clamping face of the clamping element and a clamping face of the base element.

The pinching of the infusion line takes place by sliding the clamping element onto the infusion line such that the infusion line comes to rest in between the clamping face of the clamping element and the clamping face of the base element. The clamping element hence is moved with respect to the base element such that the clamping element slides onto the infusion line and in this way pinches of the infusion line in the clamping position.

Because of the sliding movement of the clamping element a relatively small force for actuating the clamping element is sufficient, nevertheless reliably achieving a pinching of the infusion line.

For in turn releasing the infusion line, the clamping element may be moved with respect to the base element in order to slide the clamping element off the infusion line such that the pinching of the infusion line is released and a fluid may flow through the infusion line in the release position.

The clamping of the infusion line hence takes place by means of the clamping element in a sliding fashion. The clamping element may be moved with respect to the base element in a tangential fashion such that the clamping element slides onto the infusion line while the base element remains stationary (except for possibly an adjustment movement of the base element for adjusting to tolerances in a direction perpendicular to the movement direction of the clamping element) during movement of the clamping element.

In one embodiment, the clamping face of the clamping element and the clamping face of the base element extend in parallel to each other in the clamping position of the clamping element. The clamping faces receive the infusion line in between themselves, wherein the distance between the clamping faces is so small that the infusion line is reliably pinched off when the clamping element is in the clamping position.

In one embodiment, the second clamping face extends, when viewed in a cross-sectional plane perpendicular to the pivot axis of the clamping element, along an extension direction, the clamping element being movable with respect to the base element for approaching the clamping face of the clamping element towards the clamping face of the base element along the extension direction. The movement direction of the clamping element hence corresponds to the extension direction of the base element, such that the clamping element is tangentially moved with respect to the base element in order to slide the clamping element onto the infusion line for pinching off the infusion line in between the base element and the clamping element in the clamping position.

The clamping face of the clamping element and/or the clamping face of the base element are curved, the curvature of the clamping faces conforming to a circumferential direction about the pivot axis of the clamping element. The radius of the curvature of the clamping face of the base element as well as the curvature of the radius of the clamping face of the clamping element hence may be defined by the distance of the respective clamping face to the pivot axis of the clamping element, wherein due to the slightly different distances the radius of curvature of the clamping face of the clamping element may slightly differ from the radius of curvature of the clamping face of the base element.

In one embodiment, the clamping element comprises a body and a jaw element pivotably arranged on the body, the jaw element forming the clamping face of the clamping element. The body of the clamping element is pivotably connected to the housing about the pivot axis of the clamping element. The jaw element herein at least by some margin is movable with respect to the body and may in one embodiment be pretensioned with respect to the body by means of an elastic pretensioning element, for example a spring element, a movement of the jaw element with respect to the body of the clamping element hence allowing for a compensation of tolerances in the system of the infusion pump, in particular the system of the clamping mechanism as well as tolerances of the infusion line, or an adjustment to infusion lines of different dimensions and different structural built.

In an analogous fashion to the jaw element of the clamping element, the base element may be pivotably connected to the housing and may, in one embodiment, be elastically pretensioned with respect to the housing by means of another elastic pretensioning element, for example in the shape of another spring element. Hence, also the base element may, in particular in a direction perpendicular to the movement direction of the clamping element, perform an adjustment movement for compensating tolerances or for adjusting to infusion lines of different dimensions and different structural built.

Because tolerances may be compensated for on the clamping element and/or the base element and an (automatic) adjustment to infusion lines of different dimensions and different structural built may be achieved, the clamping mechanism may be used in a versatile fashion for different infusion lines without the need for a particular adaption of the clamping mechanism by a user.

In one embodiment, the clamping mechanism furthermore comprises a locking element for locking the clamping element in the clamping position with respect to the housing such that the clamping mechanism is held by the locking element in its clamping position when the locking element acts together with the clamping element. The locking element may in particular serve to lock the clamping element when a closure element in the shape of a door of the infusion device is opened such that the clamping element remains in place when the closure element is opened. The locking element may be manually releasable such that a user may act onto the locking element for removing the infusion line from the clamping mechanism once the closure element in the shape of the door is opened.

When the closure element is closed the locking element, in one embodiment, does not act together with the clamping element and in particular does not lock the clamping element such that the clamping element may be moved towards the release position for conducting a regular infusion operation.

In one embodiment, the clamping mechanism comprises a spring element being formed and arranged to elastically pretension the clamping element with respect to the housing towards a position corresponding to the release position of the clamping element. The spring element, for example being formed by a leg spring, may for example be supported with one leg on the housing and with another leg on the clamping element to exert a force in between the housing and the clamping element towards the release position. The movement of the clamping element towards the release position hence is elastically supported by means of the spring element, such that the clamping element by action of the spring element may automatically be moved towards the release position. This in particular allows that a user may easily fit an infusion line into the clamping mechanism when placing the infusion line on the receptacle of the infusion device, making the placement of the infusion line on the infusion device easy and intuitive for the user.

In one embodiment, the movement of the clamping element to the clamping position may take place automatically when a closure element of the infusion device, also denoted as door, is closed. The closure element is for example pivotably arranged on the housing and, in a closed position, at least partially covers the receptacle. When closing the closure element the closure element, in one embodiment, may act onto the clamping element in order to move the clamping element to its clamping position. By acting onto the clamping element the clamping mechanism hence is actuated in order to transfer the clamping element from the release position towards the clamping position such that, when the closure element is closed, an infusion line received on the receptacle of the infusion device is automatically pinched off and a free flow condition through the infusion line is prevented.

Hence, when closing the closure element in the shape of the door of the infusion device, an infusion line received in the receptacle is automatically pinched off, hence preventing a free-flow condition and potentially allowing to conduct a pre-test such as an integrity test to check the integrity of the pumping mechanism.

To then conduct the infusion operation the clamping mechanism is opened by transferring the clamping element from the clamping position to the release position such that a medical solution may be delivered through the infusion line in a controlled fashion by means of the pump mechanism acting onto the infusion line.

In one embodiment, the closure element comprises a body and an actuation element, for example in the shape of a cam, which is movably arranged on the body. When closing the closure element the actuation element may for example act onto the clamping element such that, by action of the actuation element, the clamping mechanism is actuated and the clamping element is moved to the clamping position.

The actuation element may for example be connected with a handle which a user actuates when closing the closure element. By means of the handle the actuation element may be moved with respect to the body of the closure element, such that the actuation element when closing the closure element acts onto the clamping element for moving the clamping element from the release position to the clamping position.

In order to automatically open the clamping mechanism for an actual infusion operation (for example after a pre-test prior to the infusion operation has been conducted) the clamping mechanism may comprise an opening mechanism having for example a pin movable with respect to the housing by means of an electric motor or the like. By means of the pin the opening mechanism may act onto the clamping element in order to transfer the clamping element from the clamping position to the release position.

The clamping mechanism may in particular be non-releasably arranged on the housing such that the clamping mechanism may act onto different infusion lines received in the receptacle of the housing. Hence, the clamping element may be non-releasably connected to the housing, in contrast to arrangements in which a clamping mechanism is arranged on an infusion line and together with the infusion line is placed on an infusion device. This may allow the use of different infusion lines of different constitution and by different manufacturers on the infusion device.

An aspect of the disclosure relates to a method for operating an infusion device, the method comprising:
- receiving an infusion line in a receptacle of a housing of the infusion device for delivering a medical fluid through the infusion line received in the receptacle using a pump mechanism, and
- pivotably moving a clamping element of a clamping mechanism with respect to the housing between a clamping position for acting onto an infusion line received in the receptacle to locally pinch off the infusion line and a release position for allowing a flow through the infusion line.

Herein, the clamping element is moved with respect to a base element of the clamping mechanism arranged on the housing for sliding the clamping element onto the infusion line for pinching the infusion line in between a first clamping face of the clamping element and a second clamping face of the base element in the clamping position or for sliding the clamping element off the infusion line for allowing a flow through the infusion line in the release position.

The advantages and advantageous embodiments described above for the infusion device equally apply also to the method, such that in this regard it shall be referred to the above.

The idea underlying the invention shall subsequently be described in more detail with respect to the embodiments shown in the figures. Herein:
- Fig. 1: shows a schematic drawing of an infusion device;
- Fig. 2: shows a schematic view of a clamping mechanism of an infusion device, in a release position of a clamping element of the clamping mechanism;
- Fig. 3: shows a schematic view of the clamping mechanism, when closing a closure element in the shape of a door of the infusion device;
- Fig. 4: shows a schematic view of the clamping mechanism, in a clamping position of the clamping element when the closure element is closed;
- Fig. 5: shows a schematic view of the clamping mechanism, in the release position of the clamping element, when actuating the clamping mechanism by an opening mechanism;
- Fig. 6: shows a schematic view of the clamping mechanism, in the clamping position of the clamping element when opening the closure element;
- Fig. 7: shows a schematic view of the clamping mechanism, while unlocking the clamping element manually by a user for transferring the clamping element towards the release position; and
- Fig. 8: shows a detailed view of the clamping element and a base element of the clamping mechanism.

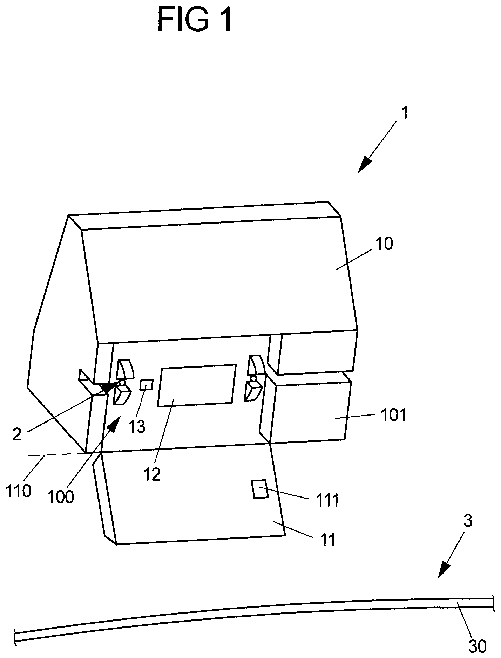

Fig. 1 shows, in a schematic view, an infusion device 1 having a housing 10 forming a receptacle 100 to receive an infusion set 3 comprising an infusion line 30 and, possibly, a pumping module in the shape of a pumping cassette or the like therein. The infusion device 1 comprises a pump mechanism 12 to, in a manner known per se, exert a pumping action onto the infusion line 30 and to in this way force a flow of a medical solution through the infusion line 30 to administer the medical solution to a patient.

It shall be noted that the infusion device 1 may be constituted to act on a pumping module of an infusion line 30 or directly on an infusion line 30. In the latter case any conventional infusion line 30 without comprising a pumping module may be used on the infusion device 1.

For conducting an infusion operation, the infusion set 3 is placed on the infusion device 1 such that it comes into operative connection with the pump mechanism 12. Upon placing the infusion set 3 on the infusion device 1, a closure element 11 in the shape of a door pivotably connected about a pivot axis 110 to the housing 10 is closed such that the infusion set 3 is securely held within the receptacle 100. During an infusion operation, then, a pressure within the infusion line 30 is monitored via a pressure sensor 13, for example to monitor a correct functioning of the device during the infusion and to possibly detect an occlusion condition stemming from an occlusion of the infusion line 30.

It shall be noted that additional pressure sensors may be present. For example, one pressure sensor may be located upstream of the pumping mechanism 12, and another pressure sensor may be located downstream of the pumping mechanism 12 in order to detect the pressure within the infusion set 3 upstream and downstream of the pumping mechanism 12.

The infusion device 1 comprises a clamping mechanism, schematically indicated at reference numeral 2 in Fig. 1, serving to pinch off the infusion line 30 upon placing the infusion line 30 in the receptacle 100 of the infusion device 1 in order to prevent a free-flow condition and to be able to for example perform a so-called occlusivity pre-test of the pumping mechanism 12.

The clamping mechanism 2, in the embodiment of Fig. 1, is arranged downstream of the pumping mechanism 12 and a pressure sensor 13, the clamping mechanism 2 being constituted to for example conduct a pressure-rise test or other tests prior to or during an infusion operation.

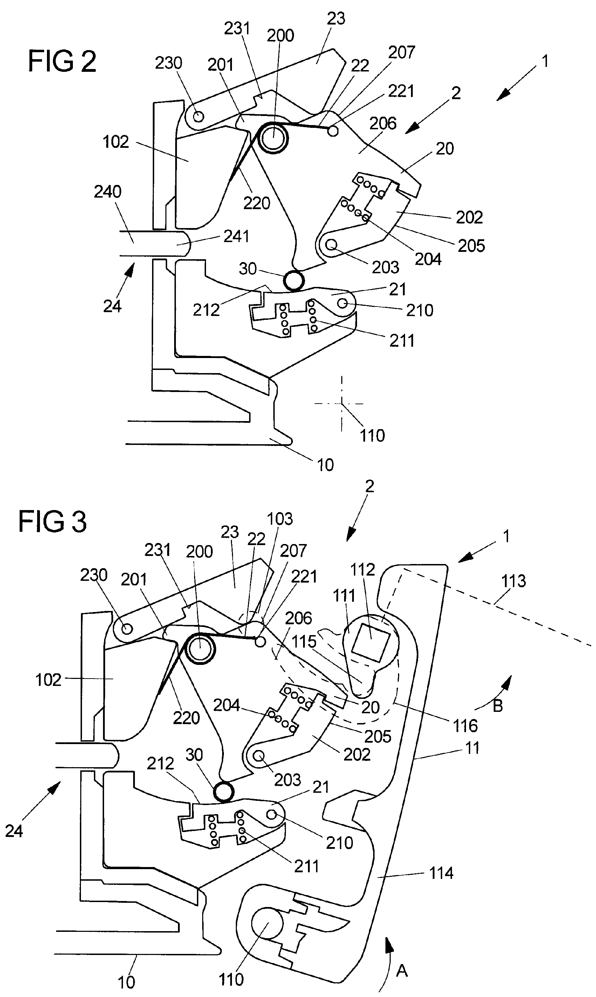

An embodiment of a clamping mechanism 2 shall subsequently be explained with reference to Figs. 2 to 8.

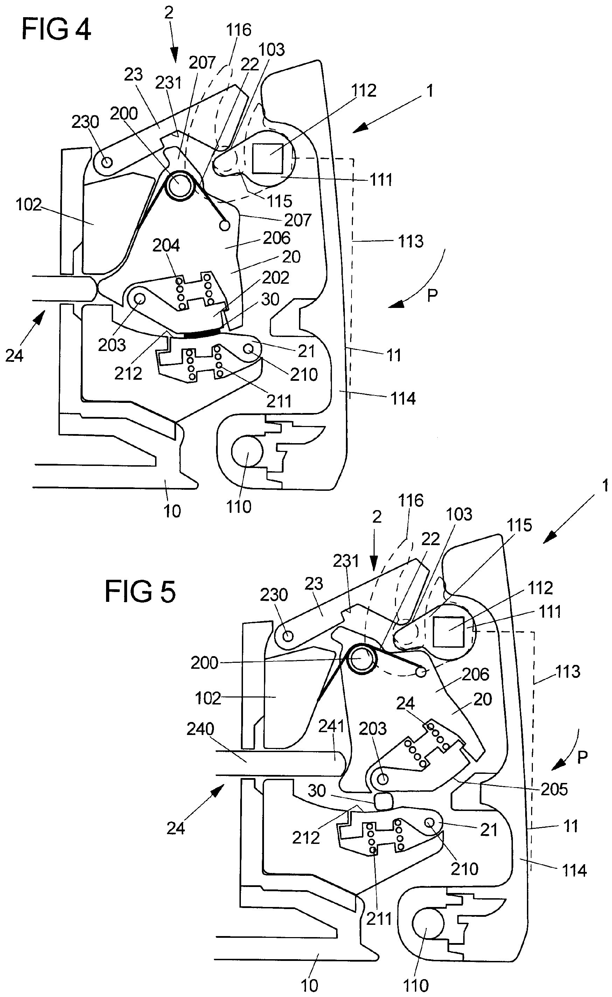

The clamping mechanism 2 of Figs. 2 to 8 comprises a clamping element 20 pivotably connected to a housing portion 102 of the housing 10 about a pivot axis 200 in a non-releasable fashion. The clamping element 20 can be moved with respect to the housing portion 102 in between a release position (Fig. 2) and a clamping position (Fig. 4) in order to, in the release position according to Fig. 2, be able to place an infusion line 30 in between the clamping element 20 and a base element 21 of the clamping mechanism 2 and, in the clamping position according to Fig. 4, locally pinch off the infusion line 30 in order to occlude the infusion line 30 to prevent a free flow of a medical solution through the infusion line 30 towards a patient.

The clamping element 20 of the clamping mechanism 2 comprises a body 206 and a jaw element 202 pivotably arranged, about a pivot axis 203, on the body 206. The body 206, about the pivot axis 200, is pivotably connected to the housing 10. The jaw element 202 can be moved relative to the body 206 about its pivot axis 203 and is elastically pretensioned with respect to the body 206 by means of a pretensioning element 204 in the shape of a spiral pressure spring or the like.

The jaw element 202 forms a clamping face 205 which, in the clamping position of the clamping mechanism 2, serves to act onto the infusion line 30 received within the clamping mechanism 2.

The clamping mechanism 2, in addition to the clamping element 20, comprises a base element 21 which, about a pivot axis 210, is pivotably arranged on the housing section 102 of the housing 10 and is elastically pretensioned with respect to the housing section 102 by means of a pretensioning element 211 in the shape of a spring element, in particular a spiral pressure spring or the like. The base element 21 forms a clamping face 212 on which the infusion line 30 is received, as shown in Fig. 2, prior to closing the clamping mechanism 2 and which acts together with the clamping face 205 of the jaw element 202 of the clamping element 20 for pinching off the infusion line 30 in the clamping position, as it is shown in Fig. 4.

The clamping element 20, together with the base element 21, serves to pinch off the infusion line 30 in a sliding fashion by slidingly moving onto the infusion line 30 when transferring the clamping element 20 from the release position (Fig. 2) to the clamping position (Fig. 4). For transferring the clamping mechanism 2 from the release position to the clamping position the clamping element 20 is moved about its pivot axis 200 and in this way is approached, with its clamping face 205 on the jaw element 202, towards the clamping face 212 of the base element 21. The approach movement herein takes place in a tangential fashion such that the jaw element 202 with its clamping face 205 slides onto the infusion line 30 placed on the base element 21 in order to pinch off the infusion line 30 in the clamping position (Fig. 4).

Both the clamping face 205 of the jaw element 202 of the clamping element 20 and the clamping face 212 of the base element 21 are, in the shown embodiment, curved about the pivot axis 200 of the clamping element 20. In the clamping position (Fig. 4) the clamping face 205 of the jaw element 202 of the clamping element 20 and the clamping face 212 of the base element 21 extend substantially parallel to each other, the infusion line 30 being received in between the clamping faces 205, 212 as shown in Fig. 4.

Because both the jaw element 202 of the clamping element 20 and the base element 21 are elastically movable with respect to the body 206 of the clamping element 20 on the one hand and with respect to the housing section 102 of the housing 10 on the other hand by means of the respective pretensioning element 204, 211, tolerances in the system as well as differences in the structural built and dimension of infusion lines 30 received in the clamping mechanism 2 may be compensated for and adjusted to. This takes place automatically in a passive fashion by an adjustment of the position of the jaw element 202 and the base element 21 with respect to each other, allowing to use different infusion lines 30 on the infusion device 1.

The clamping mechanism 2 furthermore comprises a locking element 23 pivotably arranged on the housing 10 via a pivot axis 230. The locking element 23 serves to act together with the clamping element 20 in order to lock the clamping element 20 in its clamping position, in particular in a situation in which the closure element 11 in the shape of the door is opened in order to prevent an automatic transfer of the clamping mechanism 2 from the clamping position towards the release position upon opening the closure element 11.

During regular operation of the infusion device 1 the locking element 23 is rendered inactive when the closure element 11 is closed, as shall be explained below.

The clamping mechanism 2, in an open position of the closure element 11 in the shape of the door of the infusion device 1, for example is in the release position as illustrated in Fig. 2. In the open position the closure element 11 may for example be completely open (at an angle of about 90° to the housing 10). In this state an infusion set 3 can be placed on the infusion device 1 and the infusion line 30 can be received in between the clamping element 20 and the base element 21, as shown in Fig. 2.

The clamping mechanism 2 comprises a spring element 22 in the shape of a leg spring which, with a first leg 220, is supported on the housing 10 and with another, second leg 221 is connected to the clamping element 20, the spring element 22 serving to pretension the clamping element 20 towards its release position. By means of the spring element 22 the clamping element 20 hence is held in the release position when the clamping mechanism 2 is released.

Upon closing the closure element 11 by pivoting it about its pivot axis 110 in a closing direction A the clamping mechanism 2 is automatically transferred to the clamping position of the clamping element 20, as shown in the transition from Fig. 3 to Fig. 4. Specifically, when closing the closure element 11 a user actuates a handle 113 connected to an actuation element 111 being pivotably arranged about a pivot axis 112 on a body 114 of the closure element 11, as illustrated in Fig. 3, the handle 113 being constituted to actuate a locking mechanism for locking the closure element 11 in its closed position. For closing the closure element 11 the handle 113 in particular is moved in a moving direction B, and upon closing the closure element 11 the actuation element 111 linked to the handle 113 acts onto a front face 207 of the clamping element 20 for moving it towards its clamping position.

As shown in dashed lines in Fig. 3, a detent element 116 is connected to the pivot axis 112 of the actuation element 111 such that the detent element 116 is rotated together with the actuation element 111 when actuating the handle 113. The detent element 116 serves to lock the closure element 11 with respect to the housing 10 by engaging a locking bolt 103 which is stationary with respect to the housing 10.

Both the detent element 116 and the locking bolt 103 are axially displaced with respect to the clamping mechanism 2 and the actuation element 111, such that the detent element 116 and the locking bolt 103 do not interfere with the mechanics of the clamping mechanism 2.

When closing the closure element 11 and when actuating the handle 113 as indicated in Fig. 3, the detent element 116 is in a position in which the detent element 116 may be brought into engagement with the locking bolt 103 when closing the closure element 11.

The actuation element 111 herein is in the corresponding position shown in Fig. 3, such that the actuation element 111 acts onto the front face 207 of the clamping element 20 for moving the clamping element 20 towards its clamping position as visible in the transition from Fig. 3 to Fig. 4.

When the closure element 11 has been closed, the handle 113 is rotated in a moving direction P as indicated in Fig. 4 such that the actuation element 111 is moved past the clamping element 20, and the detent element 116 is brought into locking engagement with the locking bolt 103, as this is shown in Fig. 4. The closure element 11 hence is locked with respect to the housing 10 and hence is fixedly held in its closed position.

When the closure element 11 is closed, hence, the clamping mechanism 2 is automatically actuated such that the clamping element 20 assumes the clamping position of Fig. 4. When closing the closure element 11, hence, the infusion line 30 received in the receptacle 100 of the infusion device 1 is pinched off as illustrated in Fig. 4, such that an occlusivity pre-test of the pumping mechanism may be performed.

Prior to starting an actual infusion operation, a pre-test may be conducted while the clamping mechanism 2 with its clamping element 20 is in the clamping position. In particular, a so-called pressure rise test may be conducted in order to perform a pre-test to check the integrity of the pumping mechanism 12.

When the door is closed and when the actuation element 111 is moved to the non-actuated position of Fig. 4, the actuation element 111 acts onto the locking element 23, as this can be seen from Fig. 4. In this way the locking element 23 is lifted off the clamping element 20 such that a locking recess 231 of the locking element 23 is not in engagement with a locking nose 201 formed on the body 206 of the clamping element 20. The clamping element 20 hence can be moved in between the clamping position shown in Fig. 4 and the release position shown in Fig. 5, the locking element 23 being rendered inactive.

For conducting the actual infusion operation the clamping mechanism 2 is opened by means of an opening mechanism 24 comprising a pin 240 which, in an electric fashion, can be moved with respect to the housing portion 102 in order to act onto the clamping element 20 with a tip 241 of the pin 240 and to in this way move the clamping element 20 to the release position of Fig. 5. Liquid may hence flow through the infusion line 30 and may be delivered by the infusion device 1 towards a patient connected to the infusion line 30.

The opening of the clamping mechanism 2 is initiated by the opening mechanism 24 which, with its pin 240, serves to act onto the clamping element 20 to move the clamping element 20 towards the release position.

After termination of the infusion operation a user may again wish to open the closure element 11, for which the user may grab the handle 113 to rotate the handle 113 together with the actuation element 111 about the pivot axis 112, such that the actuation element 111 in this way acts onto the clamping element 20 to again move the clamping element 20 into the clamping position of Fig. 4. This takes place by action of the end section 115 of the actuation element 111 onto the clamping element 20, such that, when opening the closure element 11, the clamping element 20 is again brought to its clamping position.

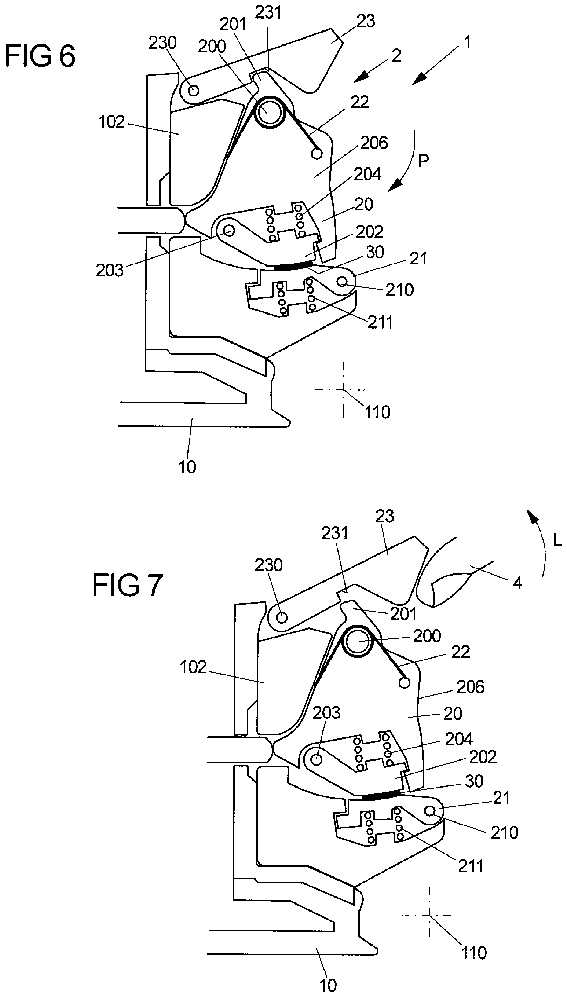

When the actuation element 111 is actuated, the end section 115 of the actuation element 111 is displaced with respect to the locking element 23 and no longer acts onto the locking element 23, such that the locking element 23, for example forced by an elastic pretensioning acting onto the locking element 23, is moved into a locking position as shown in Fig. 6. In this locking position the locking element 23 by means of its locking recess 231 engages with the locking nose 201 of the clamping element 20 such that the clamping element 20 is locked and held in the clamping position. When opening the closure element 11, hence, the clamping element 20 remains in its clamping position and cannot move towards the release position. A free flow of liquid when opening the closure element 11 in the shape of the door hence is prevented.

If a user now wishes to remove the infusion line 30 from the infusion device 1, the user, for example with a finger 4 as illustrated in Fig. 7, may act onto the locking element 23 in order to lift the locking element 23 in a lifting direction L such that the recess 231 of the locking element 23 is disengaged from the locking protrusion 201 of the clamping element 20 and the clamping element 20 may be moved towards the release position, for example by a user pulling on the infusion line 30 placed in the clamping mechanism 2.

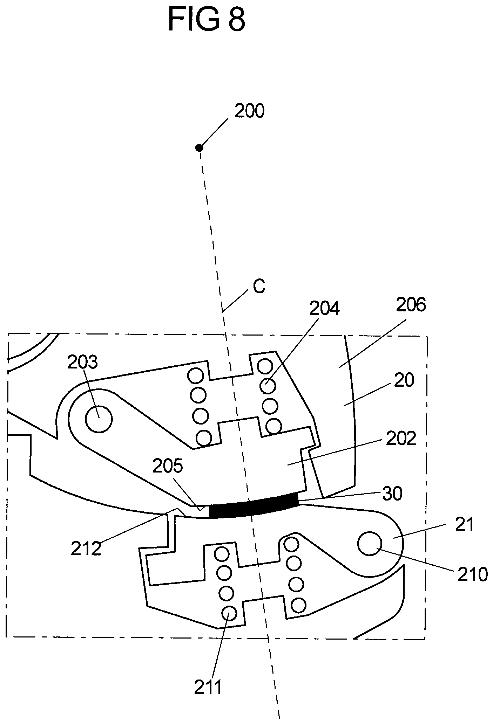

Because the jaw element 202 is movably arranged on the body 206 of the clamping element 20 and the base element 21 is movably arranged on the housing portion 102, tolerances in the system as well as differences in dimension and shape of different infusion lines 30 may be adapted to in an automatic fashion by a floating movement of the jaw element 202 and/or the base element 21 in a direction substantially perpendicular to the movement direction of the clamping element 20, i.e., substantially along a virtual line C extending through the pivot axis 200 of the clamping element 20, as illustrated in the enlarged view of Fig. 8.

The idea underlying the invention is not limited to the embodiments described above, but may be implemented in an entirely different fashion.

By means of an infusion device of the described type the placement of the infusion line on the infusion device may become simple and intuitive.

Because of the floating arrangement of the base element and the jaw element the infusion device may be compatible with a large number of different infusion lines, possibly manufactured by different manufacturers.

### List of Reference Numerals

- 1: Infusion device
- 10: Housing
- 100: Receptacle
- 101: Front face
- 102: Housing portion
- 103: Locking Bolt
- 11: Closure element (door)
- 110: Pivot axis
- 111: Actuation element (cam)
- 112: Pivot axis
- 113: Handle
- 114: Body
- 115: End section
- 116: Detent element
- 12: Pump mechanism
- 13: Sensor device
- 2: Clamp mechanism
- 20: Clamping element
- 200: Pivot axis
- 201: Locking nose
- 202: Jaw element
- 203: Pivot axis
- 204: Pretensioning element (spring element)
- 205: Clamping face
- 206: Body
- 207: Front face
- 21: Base element
- 210: Pivot axis
- 211: Pretensioning element (spring element)
- 212: Clamping face
- 22: Spring element
- 220,221: Leg
- 23: Locking element
- 230: Pivot axis

- 231: Recess
- 24: Opening mechanism
- 240: Pin
- 241: Tip
- 3: Infusion set
- 30: Line
- 4: Finger
- A: Closing direction
- B: Moving direction
- C: Line
- L: Lifting direction
- P: Pivot direction

## Claims

1. Infusion device (1), comprising:
- a housing (10) having a receptacle (100) for receiving an infusion line (30),
- a pump mechanism (12) for delivering a medical fluid through an infusion line (30) received in the receptacle (100), and
- a clamping mechanism (2) having a clamping element (20) being pivotably connected to the housing (10) about a pivot axis (200) and being movable with respect to the housing (10) between a clamping position for acting onto an infusion line (30) received in the receptacle (100) to locally pinch off the infusion line (30) and a release position for allowing a flow through the infusion line (30),
**characterized in**
**that** the clamping mechanism (2) comprises a base element (21) arranged on the housing (10), the infusion line (30) being received in between the clamping element 20 and the base element (21), the clamping element (20) being movable about its pivot axis (200) with respect to the base element (21) for sliding the clamping element (20) onto the infusion line (30) for pinching the infusion line (30) in between a first clamping face (205) of the clamping element (20) and a second clamping face (212) of the base element (21) in the clamping position or for sliding the clamping element (20) off the infusion line (30) for allowing a flow through the infusion line (30) in the release position, wherein the first clamping face (205) and/or the second clamping face (212) are curved about the pivot axis (200) of the clamping element (20) and,
wherein the clamping element (20) comprises a body (206) and a jaw element (202) pivotably arranged on the body (206), and the jaw element (202) being pretensioned with respect to the body (206) by means of an elastic pretensioning element (204) and / or wherein the base element (21) is pivotably connected to the housing (10) and is elastically pretensioned with respect to the housing (10) by means of another elastic pretensioning element (211).

2. Infusion device (1) according to claim 1, **characterized in that** the first clamping face (205) and the second clamping face (212) extend parallel to each other in the clamping position of the clamping element (20).

3. Infusion device (1) according to claim 1 or 2, **characterized in that** the second clamping face (212) extends, when viewed in a cross-sectional plane perpendicular to the pivot axis (200) of the clamping element (20), along an extension direction, the clamping element (20) being movable with respect to the base element (21) for approaching the first clamping face (205) towards the second clamping face (212) along the extension direction.

4. Infusion device (1) according to one of the preceding claims, **characterized in that** the clamping mechanism (2) comprises a locking element (23) for locking the clamping element (20) in the clamping position, the locking element (23) being manually releasable for removing the infusion line (30) from the clamping mechanism (2).

5. Infusion device (1) according to one of the preceding claims, **characterized in that** the clamping mechanism (2) comprises a spring element (22) being formed and arranged to elastically pretension the clamping element (20) with respect to the housing (10) towards a position corresponding to the release position of the clamping element (20).

6. Infusion device (1) according to one of the preceding claims, **characterized by** a closure element (11) being pivotably arranged on the housing (10) and being configured to, in a closed position, at least partially cover the receptacle (100), wherein the closure element (11) is operative to act onto the clamping element (20) for moving the clamping element (20) to its clamping position when pivoting the closure element (11) towards the closed position.

7. Infusion device (1) according to claim 6, **characterized in that** the closure element (11) comprises a body (114) and an actuation element (111) movably arranged on the body (114), the actuation element (111) being configured to act onto the clamping element (20) when pivoting the closure element (11) towards the closed position.

8. Infusion device (1) according to claim 7, **characterized in that** the closure element (11) comprises a handle (113) connected with the actuation element (111) for moving the actuation element (111) with respect to the body (114).

9. Infusion device (1) according to one of the preceding claims, **characterized in that** the clamping mechanism (2) comprises an opening mechanism (24) being configured to act onto the clamping element (20) for moving the clamping element (20) to the release position.

## Patentansprüche

1. Infusionsvorrichtung (1), die Folgendes umfasst:
- ein Gehäuse (10), das einen Behälter (100) zur Aufnahme einer Infusionsleitung (30) aufweist,
- einen Pumpenmechanismus (12) zum Abgeben eines medizinischen Fluids durch eine in dem Behälter (100) aufgenommene Infusionsleitung (30) und
- einen Klemmmechanismus (2), der ein Klemmelement (20) aufweist, das mit dem Gehäuse (10) über eine Schwenkachse (200) schwenkbar verbunden ist und in Bezug auf das Gehäuse (10) zwischen einer Klemmposition, die auf eine in dem Behälter (100) aufgenommene Infusionsleitung (30) einwirkt, um die Infusionsleitung (30) lokal abzuklemmen, und einer Freigabeposition, die einen Durchfluss durch die Infusionsleitung (30) erlaubt, beweglich ist,
**dadurch gekennzeichnet,**
**dass** der Klemmmechanismus (2) ein an dem Gehäuse (10) angeordnetes Basiselement (21) umfasst, die Infusionsleitung (30) zwischen dem Klemmelement 20 und dem Basiselement (21) aufgenommen ist, das Klemmelement (20) in Bezug auf das Basiselement (21) um seine Schwenkachse (200) beweglich ist, um das Klemmelement (20) auf die Infusionsleitung (30) zu schieben, um die Infusionsleitung (30) zwischen einer ersten Klemmfläche (205) des Klemmelements (20) und einer zweiten Klemmfläche (212) des Basiselements (21) in der Klemmposition abzuklemmen, oder das Klemmelement (20) von der Infusionsleitung (30) zu schieben, um einen Durchfluss durch die Infusionsleitung (30) in der Freigabeposition zu erlauben, wobei die erste Klemmfläche (205) und/oder die zweite Klemmfläche (212) um die Schwenkachse (200) des Klemmelements (20) gebogen ist/sind, und
wobei das Klemmelement (20) einen Körper (206) und ein an dem Körper (206) schwenkbar angeordnetes Klemmbackenelement (202) umfasst und das Klemmbackenelement (202) in Bezug auf den Körper (206) mittels eines elastischen Vorspannelements (204) vorgespannt ist und/oder wobei das Basiselement (21) schwenkbar mit dem Gehäuse (10) verbunden ist und in Bezug auf das Gehäuse (10) mittels eines weiteren elastischen Vorspannelements (211) elastisch vorgespannt ist.

2. Infusionsvorrichtung (1) nach Anspruch 1, **dadurch gekennzeichnet, dass** sich die erste Klemmfläche (205) und die zweite Klemmfläche (212) in der Klemmposition des Klemmelements (20) parallel zueinander erstrecken.

3. Infusionsvorrichtung (1) nach Anspruch 1 oder 2, **dadurch gekennzeichnet, dass** sich die zweite Klemmfläche (212) bei Betrachtung in einer Querschnittsebene senkrecht zur Schwenkachse (200) des Klemmelements (20) entlang einer Erstreckungsrichtung erstreckt, wobei das Klemmelement (20) in Bezug auf das Basiselement (21) beweglich ist, um die erste Klemmfläche (205) der zweiten Klemmfläche (212) entlang der Erstreckungsrichtung anzunähern.

4. Infusionsvorrichtung (1) nach einem der vorhergehenden Ansprüche, **dadurch gekennzeichnet, dass** der Klemmmechanismus (2) ein Verriegelungselement (23) zum Verriegeln des Klemmelements (20) in der Klemmposition umfasst, wobei das Verriegelungselement (23) zum Entfernen der Infusionsleitung (30) aus dem Klemmmechanismus (2) manuell lösbar ist.

5. Infusionsvorrichtung (1) nach einem der vorhergehenden Ansprüche, **dadurch gekennzeichnet, dass** der Klemmmechanismus (2) ein Federelement (22) umfasst, das so ausgebildet und angeordnet ist, dass es das Klemmelement (20) in Bezug auf das Gehäuse (10) in Richtung einer Position, die der Freigabeposition des Klemmelements (20) entspricht, elastisch vorspannt.

6. Infusionsvorrichtung (1) nach einem der vorhergehenden Ansprüche, **dadurch gekennzeichnet, dass** ein Verschlusselement (11) schwenkbar an dem Gehäuse (10) angeordnet ist und so konfiguriert ist, dass es den Behälter (100) in einer geschlossenen Position mindestens teilweise bedeckt, wobei das Verschlusselement (11) funktionsbereit ist, auf das Klemmelement (20) einzuwirken, um das Klemmelement (20) in seine Klemmposition zu bewegen, wenn das Verschlusselement (11) in Richtung der geschlossenen Position geschwenkt wird.

7. Infusionsvorrichtung (1) nach Anspruch 6, **dadurch gekennzeichnet, dass** das Verschlusselement (11) einen Körper (114) und ein an dem Körper (114) beweglich angeordnetes Betätigungselement (111) umfasst, wobei das Betätigungselement (111) so konfiguriert ist, dass es auf das Klemmelement (20) einwirkt, wenn das Verschlusselement (11) in Richtung der geschlossenen Position geschwenkt wird.

8. Infusionsvorrichtung (1) nach Anspruch 7, **dadurch gekennzeichnet, dass** das Verschlusselement (11) einen mit dem Betätigungselement (111) verbundenen Griff (113) zum Bewegen des Betätigungselements (111) in Bezug auf den Körper (114) umfasst.

9. Infusionsvorrichtung (1) nach einem der vorhergehenden Ansprüche, **dadurch gekennzeichnet, dass** der Klemmmechanismus (2) einen Öffnungsmechanismus (24) umfasst, der so konfiguriert ist, dass er auf das Klemmelement (20) einwirkt, um das Klemmelement (20) in die Freigabeposition zu bewegen.

## Revendications

1. Dispositif de perfusion (1), comprenant :
- un logement (10) ayant un réceptacle (100) pour recevoir une ligne de perfusion (30),
- un mécanisme de pompe (12) pour délivrer un fluide médical à travers une ligne de perfusion (30) reçue dans le réceptacle (100), et
- un mécanisme de serrage (2) ayant un élément de serrage (20) étant relié de manière à pouvoir pivoter au logement (10) autour d'un axe de pivotement (200) et pouvant se déplacer par rapport au logement (10) entre une position de serrage pour agir sur une ligne de perfusion (30) reçue dans le réceptacle (100) pour pincer localement la ligne de perfusion (30) et une position de relâchement pour permettre un écoulement à travers la ligne de perfusion (30),
**caractérisé en ce**
**que** le mécanisme de serrage (2) comprend un élément de base (21) agencé sur le logement (10), la ligne de perfusion (30) étant reçue entre l'élément de serrage 20 et l'élément de base (21), l'élément de serrage (20) pouvant se déplacer autour de son axe de pivotement (200) par rapport à l'élément de base (21) pour faire glisser l'élément de serrage (20) sur la ligne de perfusion (30) pour pincer la ligne de perfusion (30) entre une première face de serrage (205) de l'élément de serrage (20) et une seconde face de serrage (212) de l'élément de base (21) dans la position de serrage ou pour faire glisser l'élément de serrage (20) hors de la ligne der perfusion (30) pour permettre un écoulement à travers la ligne de perfusion (30) dans la position de relâchement, dans lequel la première face de serrage (205) et/ou la seconde face de serrage (212) sont incurvées autour de l'axe de pivotement (200) de l'élément de serrage (20) et,
dans lequel l'élément de serrage (20) comprend un corps (206) et un élément de mâchoire (202) agencé de manière à pouvoir pivoter sur le corps (206), et l'élément de mâchoire (202) étant précontraint par rapport au corps (206) au moyen d'un élément de précontrainte élastique (204) et / ou dans lequel l'élément de base (21) est relié de manière à pouvoir pivoter au logement (10) et est précontraint de manière élastique par rapport au logement (10) au moyen d'un autre élément de précontrainte élastique (211).

2. Dispositif de perfusion (1) selon la revendication 1, **caractérisé en ce que** la première face de serrage (205) et la seconde face de serrage (212) s'étendent parallèlement l'une à l'autre dans la position de serrage de l'élément de serrage (20).

3. Dispositif de perfusion (1) selon la revendication 1 ou 2, **caractérisé en ce que** la seconde face de serrage (212) s'étend, lorsqu'elle est vue dans un plan en section transversale perpendiculaire à l'axe de pivotement (200) de l'élément de serrage (20), le long d'une direction d'extension, l'élément de serrage (20) pouvant se déplacer par rapport à l'élément de base (21) pour approcher la première face de serrage (205) vers la seconde face de serrage (212) le long de la direction d'extension.

4. Dispositif de perfusion (1) selon l'une des revendications précédentes, **caractérisé en ce que** le mécanisme de serrage (2) comprend un élément de verrouillage (23) pour verrouiller l'élément de serrage (20) dans la position de serrage, l'élément de verrouillage (23) pouvant être relâché manuellement pour retirer la ligne de perfusion (30) du mécanisme de serrage (2).

5. Dispositif de perfusion (1) selon l'une des revendications précédentes, **caractérisé en ce que** le mécanisme de serrage (2) comprend un élément ressort (22) étant formé et agencé pour précontraindre élastiquement l'élément de serrage (20) par rapport au logement (10) vers une position correspondant à la position de relâchement de l'élément de serrage (20).

6. Dispositif de perfusion (1) selon l'une des revendications précédentes, **caractérisé par un** élément de fermeture (11) étant agencé de manière à pouvoir pivoter sur le logement (10) et étant conçu pour, dans une position fermée, recouvrir au moins partiellement le réceptacle (100), dans lequel l'élément de fermeture (11) peut fonctionner pour agir sur l'élément de serrage (20) pour déplacer l'élément de serrage (20) vers sa position de serrage lors du pivotement de l'élément de fermeture (11) vers la position fermée.

7. Dispositif de perfusion (1) selon la revendication 6, **caractérisé en ce que** l'élément de fermeture (11) comprend un corps (114) et un élément d'actionnement (111) agencé de manière à pouvoir se déplacer sur le corps (114), l'élément d'actionnement (111) étant conçu pour agir sur l'élément de serrage (20) lors du pivotement de l'élément de fermeture (11) vers la position fermée.

8. Dispositif de perfusion (1) selon la revendication 7, **caractérisé en ce que** l'élément de fermeture (11) comprend une poignée (113) reliée à l'élément d'actionnement (111) pour déplacer l'élément d'actionnement (111) par rapport au corps (114).

9. Dispositif de perfusion (1) selon l'une des revendications précédentes, **caractérisé en ce que** le mécanisme de serrage (2) comprend un mécanisme d'ouverture (24) étant conçu pour agir sur l'élément de serrage (20) pour déplacer l'élément de serrage (20) vers la position de relâchement.
